(19) **Europäisches Patentamt**

**European Patent Office**

**Office européen des brevets**

(11) **EP 1 040 814 B1**

(12) **FASCICULE DE BREVET EUROPEEN**

(45) Date de publication et mention
de la délivrance du brevet:
**03.04.2002   Bulletin 2002/14**

(51) Int Cl.⁷: **A61K 7/043**

(21) Numéro de dépôt: **00400824.9**

(22) Date de dépôt: **24.03.2000**

(54) **VERNIS A ONGLES COMPRENANT UNE DISPERSION AQUEUSE DE POLYMERE**

EINE WÄSSRIGE POLYMERDISPERSION ENTHALTENDER NAGELLACK

NAIL ENAMEL CONTAINING AN AQUEOUS POLYMER DISPERSION

(84) Etats contractants désignés:
**AT BE CH CY DE DK ES FI FR GB GR IE IT LI LU
MC NL PT SE**

(30) Priorité: **01.04.1999  FR 9904103**

(43) Date de publication de la demande:
**04.10.2000   Bulletin 2000/40**

(73) Titulaire: **L'OREAL**
**75008 Paris (FR)**

(72) Inventeurs:
• **Leuridan, Frédéric**
**75005 Paris (FR)**
• **Colombel, Dolorès**
**94240 L'Hay-les-Roses (FR)**
• **Lion, Bertrand**
**95270 Luzarches (FR)**

(74) Mandataire: **Kromer, Christophe**
**L'OREAL - D.P.I.,**
**6, rue Bertrand Sincholle**
**92585 Clichy Cedex (FR)**

(56) Documents cités:
**WO-A-97/00664**          **FR-A- 2 739 022**
**US-A- 5 607 665**          **US-A- 5 639 447**

• **PATENT ABSTRACTS OF JAPAN vol. 1998, no.
06, 30 avril 1998 (1998-04-30) & JP 10 036227 A
(POLA CHEM IND INC), 10 février 1998
(1998-02-10)**
• **PATENT ABSTRACTS OF JAPAN vol. 1995, no.
07, 31 août 1995 (1995-08-31) & JP 07 089827 A
(KAO CORP), 4 avril 1995 (1995-04-04)**

**Description**

La présente invention a pour objet une composition de vernis ou de soin des ongles, comprenant une dispersion aqueuse de particules de polymère, un agent plastifiant et un agent de coalescence particuliers. L'invention a aussi pour objet un procédé de maquillage et/ou de soin des ongles, notamment des ongles d'êtres humains ou des faux ongles.

La composition de vernis ou de soin des ongles peut être employée comme base pour vernis, comme produit de maquillage des ongles, comme composition de finition, encore appelée "top-coat" en terminologie anglosaxonne, à appliquer sur le produit de maquillage des ongles ou bien encore comme produit de soin cosmétique des ongles.

Il est connu des compositions de vernis à ongles comprenant des dispersions aqueuse de particules de polymère filmogène. Les propriétés de ces vernis ne sont pas toujours satisfaisantes ; en particulier, le film peut présenter une mauvaise adhérence sur l'ongle et/ou ne pas être suffisamment brillant. De plus, le vernis, après séchage, est souvent difficile à démaquiller, même avec les dissolvants classiques à base d'acétone ou d'acétate d'éthyle par exemple.

Le but de la présente invention est de proposer un vernis à ongles à milieu aqueux présentant de bonnes propriétés telles que l'adhérence sur l'ongle et la brillance, et une aptitude à se démaquiller avec les dissolvants classiques à base d'acétone et/ou d'acétate d'éthyle.

Le demandeur a constaté qu'un tel vernis à ongles pouvait être obtenu en employant un polymère en dispersion aqueux particulier associé à des solvants sélectionnés.

Plus précisément, l'invention a pour objet une composition de vernis ou de soin des ongles comprenant une dispersion aqueuse de particules de polymère, caractérisée par le fait que :

-   le polymère est un polymère acrylique ayant au moins une température de transition vitreuse (Tg) supérieure ou égale à 45 °C et une température minimale de filmification (TMF) telle que Tg - TMF $\leq$ 20 °C,
    et que la composition comprend, en outre :

-   au moins un premier solvant organique ayant un point d'ébullition, mesuré à pression ambiante, supérieur ou égal à 225 °C, et
-   au moins un deuxième solvant organique ayant un point d'ébullition, mesuré à pression ambiante, allant de 70 °C à 180 °C.

L'invention a également pour objet un procédé cosmétique de maquillage et/ou de soin des ongles, consistant à appliquer sur les ongles une composition telle que définie précédemment.

L'invention a encore pour objet l'utilisation dans une composition de vernis ou de soin des ongles d'un polymère acrylique en dispersion aqueuse, ayant au moins une température de transition vitreuse (Tg) supérieure ou égale à 45 °C et une température minimale de filmification (TMF) telle que Tg - TMF $\leq$ 20 °C, d'un premier solvant organique ayant un point d'ébullition, mesuré à pression ambiante, supérieur ou égal à 225 °C, et d'un deuxième solvant organique ayant un point d'ébullition, mesuré à pression ambiante, allant de 70 °C à 180 °C, pour l'obtention d'un film démaquillable à l'acétone et/ou à l'acétate d'éthyle et/ou adhérent sur l'ongle et/ou brillant.

Avantageusement, le polymère acrylique a au moins une température de transition vitreuse (Tg) supérieure ou égale à 45 °C et une température minimale de filmification (TMF) telle que Tg - TMF $\leq$ 10 °C, et mieux $\leq$ 5 °C .

De préférence, le polymère en dispersion aqueuse a une température Tg inférieure à 70 °C, et mieux qui va de 55 °C à 65 °C. La mesure de la température de transition vitreuse (Tg) est effectuée par DSC (Differential Scanning Calorimetry) selon la norme ASTM D3418-97.

Le polymère acrylique peut être un copolymère styrène/acrylate, et notamment un polymère choisi parmi les copolymères issus de la polymérisation d'au moins un monomère styrénique et au moins un monomère (méth)acrylate d'alkyle en $C_1$-$C_{18}$.

Comme monomère styrénique utilisable dans l'invention, on peut citer le styrène ou l'alpha-méthylstyrène, et de préférence le styrène.

Le monomère de (méth)acrylate d'alkyle en $C_1$-$C_{18}$ est de préférence un (méth)acrylate d'alkyle en $C_1$-$C_{12}$ et mieux un (méth)acrylate d'alkyle en $C_1$-$C_{10}$.

Le monomère (méth)acrylate d'alkyle en $C_1$-$C_{18}$ peut être choisi parmi l'acrylate de méthyle, le méthacrylate de méthyle l'acrylate d'éthyle, l'acrylate de propyle, l'acrylate de butyle, le méthacrylate de butyle, l'acrylate d'hexyle, l'acrylate d'octyle, l'acrylate de 2-éthyle hexyle, le (méth)acrylate de lauryle, le (méth)acrylate de stéaryle.

Avantageusement, le polymère acrylique en dispersion aqueuse présente des propriétés de solubilité à 25 °C dans des solvants organiques correspondant aux paramètres moyens de solubilité dD, dP, et dH de HANSEN satisfaisant aux conditions suivantes :

-   dD = 17,5
-   dP = 7
-   dH = 7,6

avec un rayon R allant de 5 à 10, et de préférence de 5 à 6.

La définition des solvants dans l'espace de solubilité tridimensionnel selon HANSEN est décrite dans l'article de C. M. HANSEN : « The three dimensionnal solubility parameters » J. Paint Technol. 39, 105 (1967).

- dD caractérise les forces de dispersion de LONDON issues de la formation de dipôles induits lors des chocs moléculaires.
- dP caractérise les forces d'intéraction de DEBYE entre dipôles permanents ainsi que les forces d'intéractions de KEESOM entre dipôles induits et dipôles permanents.
- dH caractérise les forces d'intéractions spécifiques (type liaison hydrogène, acide/base, donneur/accepteur, etc...)
- Les paramètres dD, dP, dH sont exprimés en $(J/cm^3)^{1/2}$.

Le rayon R correspond à la distance séparant, dans l'espace des paramètres de solubilité de Hansen, un solvant organique du point dudit espace corespondant à dD = 17,5 ; dP = 7 ; dH = 7,6, R vérifiant la relation suivante :

$$5 \ J^{1/2}cm^{-3/2} \leq R \leq 10 \ J^{1/2}cm^{-3/2},$$

dans laquelle :

$$R = \sqrt{4(\delta^s_d - 17,5)^2 + (\delta^s_p - 7)^2 + (\delta^s_h - 7,6)^2}$$

et où $\delta^s_d$, $\delta^s_p$, $\delta^s_h$ sont les paramètres de solubilité de Hansen d'un solvant organique pour lequel le polymère acrylique utilisé dans la présente invention présente des propriétés de solubilité. La définition du rayon R est connue de l'ouvrage de Allan F. M. Barton, CRC Handbook of solubility parameters and other cohesion parameters, Second edition, 1991, pages 95 à 109.

Comme polymère acrylique en dispersion aqueuse, on peut utiliser selon l'invention le copolymère styrène/acrylate commercialisé sous la dénomination « JONCRYL SCX-8211 » par la société Johnson.

Selon un mode de réalisation particulier de l'invention, la composition peut comprendre comme unique polymère en dispersion aqueuse le polymère acrylique défini précédemment.

Le polymère acrylique en dispersion aqueuse peut être présent en une teneur, en matière sèche, efficace pour former un film, notamment en une teneur allant de 3 % à 50 % en poids, par rapport au poids total de la composition, et mieux de 10 % à 40 % en poids.

Le premier solvant organique présent dans la composition, appelé également plastifiant, permet de plastifier le polymère en dispersion aqueuse. De préférence, le premier solvant organique peut avoir un coefficient de partage D inférieur ou égal à 0,1. Le coefficient de partage est déterminé conformément à l'enseignement du document publié dans la revue "Progress in Organic Coatings, vol 30, 1997, pp 173-177 intitulé "a method to predict the distribution coefficient of coalescing agents between latex particles and the water phase".

Le premier solvant organique selon l'invention peut être est choisi parmi l'adipate de diisobutyle, l'ester de l'acide tertio-butyrique et du tri-méthyl-2,2,4 pentane-diol-1,3, l'adipate de diéthyle, le phtalate de diéthyle, le phtalate de dibutyle, le phtalate de dioctyle, le phtalate de butyle et de 2-éthyl hexyle, le sébacate de diméthyle, le sébacate de dibutyle, le stéarate d'éthyle, le palmitate de 2-éthyl hexyle, le n-butyl éther de dipropylène glycol, et leurs mélanges.

Avantageusement, le premier solvant organique peut être choisi parmi l'adipate de diisobutyle, l'ester de l'acide tertio-butyrique et du tri-méthyl-2,2,4 pentane-diol-1,3, le n-butyl éther de dipropylène glycol, et leurs mélanges.

Préférentiellement, le premier solvant organique a un point d'ébullition, mesuré à pression ambiante, supérieur ou égal à 230°C et inférieur ou égal à 285 °C, de préférence inférieur ou égal à 270 °C et mieux inférieur ou égal à 250 °C. Dans la présente demande, les valeurs de point d'ébullition sont à considérer à ± 2 °C en raison des incertitudes de la mesure du point d'ébullition.

Le premier solvant organique peut être présent dans la composition selon l'invention en une teneur allant de 0,1 % à 20 % en poids, par rapport au poids total de la composition, et mieux de 0,5 % à 10 %.

Le deuxième solvant organique présent dans la composition, appelé encore agent de coalescence, favorise la coalescence des particules de polymère en dispersion aqueuse. De préférence, le deuxième solvant organique peut avoir un coefficient de partage D' supérieur ou égal à 0,5, mesuré selon l'enseignement de la revue citée précédemment.

Comme deuxième solvant organique, on peut utiliser selon l'invention le n-butyl éther de propylène glycol, le diméthyl éther de dipropylène glycol, l'acétate du méthyl éther de propylène glycol, le propyl éther de propylène glycol, le lactate de méthyle, le lactate d'éthyle, le lactate d'isopropyle, et leurs mélanges.

De préférence, lè deuxième solvant organique est choisi parmi le n-butyl éther de propylène glycol, le diméthyl éther de dipropylène glycol, le lactate d'isopropyle, et leurs mélanges.

Préférentiellement, le deuxième solvant organique a un point d'ébullition, mesuré à pression ambiante, allant de 90 °C à 180 °C, et mieux de 150 °C à 180 °C.

Le deuxième solvant organique peut être présent dans la composition en une teneur allant de 2 % à 15 % en poids, par rapport au poids total de la composition, et de préférence de 3 % à 10 %.

La composition de l'invention peut comprendre, en outre, au moins un additif choisi dans le groupe formé par les agents épaississants, les agents d'étalement, les agents mouillants, les agents dispersants, les anti-mousses, les conservateurs, les filtres UV, les colorants, les pigments, les actifs, les tensioactifs, les agents hydratants, les parfums, les neutralisants, les stabilisants, les antioxydants, et leurs associations.

L'invention est illustrée plus en détail dans les exemples suivants :

**Exemple 1 :**

On a préparé un vernis à ongles ayant la composition suivante :

- Polymère acrylate/styrène en dispersion aqueuse à 44 % de matières sèches (JONCRYL SCX-8211 de la société Johnson)       28,8 g MA
- Adipate de di-isobutyle       2,8 g
- Propylène glycol de n-butyl éther       2,7 g
- Dipropylène glycol de n-butyl éther       1,6 g
- Laponite XLS       0,8 g
- Pigments       2 g
- Eau       qsp 100 g

Le vernis s'applique facilement et conduit, après séchage, à un film brillant, adhérant bien sur l'ongle et démaquillable avec de l'acétone ou de l'acétate d'éthyle.

**Exemple 2 :**

On a préparé un vernis à ongles ayant la composition suivante :

- Polymère acrylate/styrène en dispersion aqueuse à 44 % de matières sèches (JONCRYL SCX-8211 de la société Johnson)       30 g MA
- Ester de l'acide tertio-butylique et du tri-méthyl-2,2,4 pentane-diol-1,3       2,8 g
- Propylène-glycol de n-butyl éther       2 g
- Dipropylène glycol de n-butyl éther       1,6 g
- Laponite XLS       0,8 g
- Pigments       2 g
- Eau       qsp 100 g

Le vernis s'applique facilement et conduit, après séchage, à un film brillant, adhérant bien sur l'ongle et démaquillable avec de l'acétone ou de l'acétate d'éthyle.

**Revendications**

1. Composition de vernis ou de soin des ongles comprenant une dispersion aqueuse de particules de polymère, **caractérisée par le fait que** :

   - le polymère est un polymère acrylique ayant au moins une température de transition vitreuse (Tg) supérieure ou égale à 45 °C et une température minimale de filmification (TMF) telle que $Tg - TMF \leq 20$ °C,
   - et que la composition comprend, en outre :

     - au moins un premier solvant organique ayant un point d'ébullition, mesuré à pression ambiante, supérieur ou égal à 225 °C, et
     - au moins un deuxième solvant organique ayant un point d'ébullition, mesuré à pression ambiante, allant de 70 °C à 180 °C.

2. Composition selon la revendication 1, **caractérisée par le fait que** le polymère acrylique a au moins une température de transition vitreuse (Tg) supérieure ou égale à 45 °C et une température minimale de filmification (TMF) telle que $Tg - TMF \leq 10$°C.

3. Composition selon la revendication 1 ou 2, **caractérisée par le fait que** le polymère acrylique a au moins une température de transition vitreuse (Tg) supérieure ou égale à 45 °C et une température minimale de filmification (TMF) telle que $Tg - TMF \leq 5$ °C.

4. Composition selon l'une quelconque des revendications précédentes, **caractérisée par le fait que** la température de transition vitreuse Tg est inférieure à 70 °C.

5. Composition selon l'une quelconque des revendications précédentes, **caractérisée par le fait que** la température de transition vitreuse Tg va de 55 °C à 65 °C.

6. Composition selon l'une quelconque des revendications précédentes, **caractérisée par le fait que** le polymère acrylique est un copolymère styrène/acrylate.

7. Composition selon la revendication 6, **caractérisée par le fait que** le copolymère styrène/acrylate comprend au moins un monomère (méth)acrylate d'alkyle en $C_1$-$C_{18}$.

8. Composition selon la revendication 7, **caractérisée par le fait que** le copolymère styrène/acrylate comprend au moins un monomère acrylate d'alkyle en $C_1$-$C_{10}$.

9. Composition selon l'une quelconque des revendications précédentes, **caractérisée par le fait que** le polymère en dispersion aqueuse consiste essentiellement en ledit polymère acrylique.

10. Composition selon l'une quelconque des revendications précédentes, **caractérisée par le fait que** le polymère acrylique est présent, en matière sèche, en une teneur allant de 3 % à 50 % en poids, par rapport au poids total de la composition, de préférence de 10 % à 40 %.

**11.** Composition selon l'une quelconque des revendications précédentes, **caractérisée par le fait que** le premier solvant organique a un point d'ébullition, mesuré à pression ambiante, supérieur ou égal à 230 °C.

**12.** Composition selon l'une quelconque des revendications précédentes, **caractérisée par le fait que** le premier solvant organique a un coefficient de partage D inférieur ou égal à 0,1.

**13.** Composition selon l'une quelconque des revendications précédentes, **caractérisée par le fait que** le premier solvant organique est choisi dans le groupe formé par l'adipate de diisobutyle, l'ester de l'acide tertio-butyrique et du tri-méthyl-2,2,4 pentane-diol-1,3, l'adipate de diéthyle, le phtalate de diéthyle, le phtalate de dibutyle, le phtalate de dioctyle, le phtalate de butyle et de 2-éthyl hexyle, le sébacate de diméthyle, le sébacate de dibutyle, le stéarate d'éthyle, le palmitate de 2-éthyl hexyle, le n-butyl éther de dipropylène glycol, et leurs mélanges.

**14.** Composition selon l'une quelconque des revendications précédentes, **caractérisée par le fait que** le premier solvant organique est choisi dans le groupe formé par l'adipate de diisobutyle, l'ester de l'acide tertio-butyrique et du tri-méthyl-2,2,4 pentane-diol-1,3, le n-butyl éther de dipropylène glycol, et leurs mélanges.

**15.** Composition selon l'une quelconque des revendications précédentes, **caractérisée par le fait que** le premier solvant organique est présent en une teneur allant de 0,1 % à 20 % en poids, par rapport au poids total de la composition, de préférence de 0,5 % à 10 %.

**16.** Composition selon l'une quelconque des revendications précédentes, **caractérisée par le fait que** le deuxième solvant organique a un coefficient de partage D' supérieur ou égal à 0,5.

**17.** Composition selon l'une quelconque des revendications précédentes, **caractérisée par le fait que** le deuxième solvant organique est choisi dans le groupe formé par le n-butyl éther de propylène glycol, le diméthyl éther de dipropylène glycol, l'acétate du méthyl éther de propylène glycol, le propyl éther de propylène glycol, le lactate de méthyle, le lactate d'éthyle, le lactate d'isopropyle, et leurs mélanges.

**18.** Composition selon l'une quelconque des revendications précédentes, **caractérisée par le fait que** le deuxième solvant organique est choisi dans le groupe formé par le n-butyl éther de propylène glycol, le diméthyl éther de dipropylène glycol, le lactate d'isopropyle, et leurs mélanges.

**19.** Composition selon l'une quelconque des revendications précédentes, **caractérisée par le fait que** le deuxième solvant organique est présent en une teneur allant de 2 % à 15 % en poids, par rapport au poids total de la composition, et de préférence de 3 % à 10 %.

**20.** Composition selon l'une quelconque des revendications précédentes, **caractérisée par le fait qu'**elle comprend en outre au moins un additif choisi dans le groupe formé par les agents épaississants, les agents d'étalement, les agents mouillants, les agents dispersants, les anti-mousses, les conservateurs, les filtres UV, les colorants, les pigments, les actifs, les tensioactifs, les agents hydratants, les parfums, les neutralisants, les stabilisants, les antioxydants.

**21.** Procédé cosmétique de maquillage et/ou de soin des ongles, consistant à appliquer sur les ongles une composition selon l'une quelconque des revendications 1 à 20.

**22.** Utilisation dans une composition de vernis ou de soin des ongles d'un polymère acrylique en dispersion aqueuse, ayant une température de transition vitreuse (Tg) supérieure ou égale à 45 °C et une température minimale de filmification (TMF) telle que Tg - TMF ≤ 20 °C, d'un premier solvant organique ayant un point d'ébullition, mesuré à pression ambiante, supérieur ou égal à 225 °C, et d'un deuxième solvant organique ayant un point d'ébullition, mesuré à pression ambiante, allant de 70 °C à 180 °C, pour l'obtention d'un film démaquillable à l'acétone et/ou à l'acétate d'éthyle et/ou adhérent sur l'ongle et/ou brillant.

**Claims**

**1.** Composition for varnishing or caring for the nails comprising an aqueous dispersion of polymer particles, **characterized in that** :

- the polymer is an acrylic polymer having at least one glass transition temperature (Tg) of greater than or equal to 45°C and a minimum film-forming temperature (MFT) such that Tg - MFT ≤ 20°C,
- and **in that** the composition additionally comprises:

  - at least one first organic solvent having a boiling point, measured at ambient pressure, of greater than or equal to 225°C, and

- at least one second organic solvent having a boiling point, measured at ambient pressure, ranging from 70°C to 180°C.

2. Composition according to Claim 1, **characterized in that** the acrylic polymer has at least one glass transition temperature (Tg) of greater than or equal to 45°C and a minimum film-forming temperature (MFT) such that Tg - MFT ≤ 10°C.

3. Composition according to Claim 1 or 2, **characterized in that** the acrylic polymer has at least one glass transition temperature (Tg) of greater than or equal to 45°C and a minimum film-forming temperature (MFT) such that Tg - MFT ≤ 5°C.

4. Composition according to any one of the preceding claims, **characterized in that** the glass transition temperature Tg is less than 70°C.

5. Composition according to any one of the preceding claims, **characterized in that** the glass transition temperature Tg ranges from 55°C to 65°C.

6. Composition according to any one of the preceding claims, **characterized in that** the acrylic polymer is a styrene/acrylate copolymer.

7. Composition according to Claim 6, **characterized in that** the styrene/acrylate copolymer comprises at least one $C_1$-$C_{18}$ alkyl (meth)acrylate monomer.

8. Composition according to Claim 7, **characterized in that** the styrene/acrylate copolymer comprises at least one $C_1$-$C_{10}$ alkyl acrylate monomer.

9. Composition according to any one of the preceding claims, **characterized in that** the polymer in aqueous dispersion is composed essentially of the said acrylic polymer.

10. Composition according to any one of the preceding claims, **characterized in that** the acrylic polymer is present, on a dry basis, in a content ranging from 3% to 50% by weight with respect to the total weight of the composition, preferably from 10% to 40%.

11. Composition according to any one of the preceding claims, **characterized in that** the first organic solvent has a boiling point, measured at ambient pressure, of greater than or equal to 230°C.

12. Composition according to any one of the preceding claims, **characterized in that** the first organic solvent has a partition coefficient D of less than or equal to 0.1.

13. Composition according to any one of the preceding claims, **characterized in that** the first organic solvent is chosen from the group formed by diisobutyl adipate, the ester of tert-butyric acid and of 2,2,4-trimethyl-1,3-pentanediol, diethyl adipate, diethyl phthalate, dibutyl phthalate, dioctyl phthalate, butyl 2-ethylhexyl phthalate, dimethyl sebacate, dibutyl sebacate, ethyl stearate, 2-ethylhexyl palmitate, dipropylene glycol n-butyl ether, and their mixtures.

14. Composition according to any one of the preceding claims, **characterized in that** the first organic solvent is chosen from the group formed by diisobutyl adipate, the ester of tert-butyric acid and of 2,2,4-trimethyl-1,3-pentanediol, dipropylene glycol n-butyl ether, and their mixtures.

15. Composition according to any one of the preceding claims, **characterized in that** the first organic solvent is present in a content ranging from 0.1% to 20% by weight with respect to the total weight of the composition, preferably from 0.5% to 10%.

16. Composition according to any one of the preceding claims, **characterized in that** the second organic solvent has a partition coefficient D' of greater than or equal to 0.5.

17. Composition according to any one of the preceding claims, **characterized in that** the second organic solvent is chosen from the group formed by propylene glycol n-butyl ether, dipropylene glycol dimethyl ether, propylene glycol methyl ether acetate, propylene glycol propyl ether, methyl lactate, ethyl lactate, isopropyl lactate, and their mixtures.

18. Composition according to any one of the preceding claims, **characterized in that** the second organic solvent is chosen from the group formed by propylene glycol n-butyl ether, dipropylene glycol dimethyl ether, isopropyl lactate, and their mixtures.

19. Composition according to any one of the preceding claims, **characterized in that** the second organic solvent is present in a content'ranging from 2% to 15% by weight with respect to the total weight of the composition and preferably from 3% to 10%.

20. Composition according to any one of the preceding claims, **characterized in that** it additionally comprises at least one additive chosen from the group formed by thickening agents, spreading agents, wetting agents, dispersing agents, antifoaming agents, preservatives, UV screening agents, dyes, pigments, active principles, surfactants, moisturizing agents, fragrances, neutralizing agents, stabilizing agents and antioxidants.

21. Cosmetic process for making up and/or caring for the nails, which consists in applying, to the nails, a composition according to any one of Claims 1 to 20.

22. Use in a composition for varnishing or caring for the nails of an acrylic polymer in aqueous dispersion having a glass transition temperature (Tg) of greater than or equal to 45°C and a minimum film-forming temperature (MFT) such that Tg - MET $\leq$ 20°C, of a first organic solvent having a boiling point, measured at ambient pressure, of greater than or equal to 225°C and of a second organic solvent having a boiling point, measured at ambient pressure, ranging from 70°C to 180°C, for producing a film which can be removed with acetone and/or with ethyl acetate and/or which adheres to the nail and/ or is glossy.

**Patentansprüche**

1. Zusammensetzung zum Lackieren oder zur Pflege der Nägel, die eine wässerige Dispersion von Polymerpartikeln enthält, **dadurch gekennzeichnet, dass**:

   - es sich bei dem Polymer um ein Acrylpolymer handelt, das zumindest eine Glasübergangstemperatur (Tg) von größer oder gleich 45 °C und eine minimale Temperatur für die Filmbildung (TMF) aufweist, die so ist, dass Tg - TMF $\leq$ 20 °C ist,
   - und dass die Zusammensetzung ferner

      - mindestens ein erstes organisches Lösemittel mit einem bei atmosphärischem Druck bestimmten Siedepunkt von größer oder gleich 225 °C und
      - mindestens ein zweites organisches Lösemittel mit einem bei atmosphärischem Druck bestimmten Siedepunkt im Bereich von 70 bis 180 °C

   enthält.

2. Zusammensetzung nach Anspruch 1, **dadurch gekennzeichnet, dass** das Acrylpolymer zumindest eine Glasübergangstemperatur (Tg) von größer oder gleich 45 °C und eine minimale Temperatur für die Filmbildung (TMF) aufweist, die so ist, dass Tg - TMF $\leq$ 10 °C ist.

3. Zusammensetzung nach Anspruch 1 oder 2, **dadurch gekennzeichnet, dass** das Acrylpolymer zumindest eine Glasübergangstemperatur (Tg) von größer oder gleich 45 °C und eine minimale Temperatur für die Filmbildung (TMF) aufweist, die so ist, dass Tg - TMF $\leq$ 5 °C ist.

4. Zusammensetzung nach einem der vorhergehenden Ansprüche, **dadurch gekennzeichnet, dass** die Glasübergangstemperatur Tg kleiner 70 °C ist.

5. Zusammensetzung nach einem der vorhergehenden Ansprüche, **dadurch gekennzeichnet, dass** die Glasübergangstemperatur Tg im Bereich von 55 bis 65 °C liegt.

6. Zusammensetzung nach einem der vorhergehenden Ansprüche, **dadurch gekennzeichnet, dass** das Acrylpolymer ein Styrol/Acrylat-Copolymer ist.

7. Zusammensetzung nach Anspruch 6, **dadurch gekennzeichnet, dass** das Styrol/Acrylat-Copolymer mindestens ein $C_{1-18}$-Alkyl(meth)acrylat-Monomer enthält.

8. Zusammensetzung nach Anspruch 7, **dadurch gekennzeichnet, dass** das Styrol/Acrylat-Copolymer mindestens ein $C_{1-10}$-Alkylacrylat-Monomer enthält.

9. Zusammensetzung nach einem der vorhergehenden Ansprüche, **dadurch gekennzeichnet, dass** das Polymer in wässeriger Dispersion im wesentlichen aus dem Acrylpolymer besteht.

10. Zusammensetzung nach einem der vorhergehenden Ansprüche, **dadurch gekennzeichnet, dass** das Acrylpolymer in einem als Trockensubstanz ausgedrückten Mengenanteil von 3 bis 50 Gew.-% und vorzugsweise von 10 bis 40 Gew.-%, bezogen auf das Gesamtgewicht der Zusammensetzung, vorliegt.

11. Zusammensetzung nach einem der vorhergehenden Ansprüche, **dadurch gekennzeichnet, dass** das erste organische Lösemittel einen bei atmosphärischem Druck bestimmten Siedepunkt von größer oder gleich 230 °C aufweist.

12. Zusammensetzung nach einem der vorhergehenden Ansprüche, **dadurch gekennzeichnet, dass** das erste organische Lösemittel einen Verteilungskoeffizienten D von kleiner oder gleich 0,1 aufweist.

13. Zusammensetzung nach einem der vorhergehenden Ansprüche, **dadurch gekennzeichnet, dass** das erste organische Lösemittel unter Diisobutyladipat, dem Ester von *tert*.-Butansäure und 2,2,4-Trimethylpentan-1,3-diol, Diethyladipat, Diethylphthalat, Dibutylphthalat, Dioctylphthalat, Butyl-2-ethylhexylphthalat, Dimethylsebacat, Dibutylsebacat, Ethylstearat, 2-Ethylhexylpalmitat, Dipropylenglykol-n-butylether und den Gemischen dieser Verbindungen ausgewählt ist.

**14.** Zusammensetzung nach einem der vorhergehenden Ansprüche, **dadurch gekennzeichnet, dass** das erste organische Lösemittel unter Diisobutyladipat, dem Ester von *tert*.-Butansäure und 2,2,4-Trimethylpentan-1,3-diol, Dipropylenglykol-n-butylether und den Gemischen dieser Verbindungen ausgewählt ist.

**15.** Zusammensetzung nach einem der vorhergehenden Ansprüche, **dadurch gekennzeichnet, dass** das erste organische Lösemittel in einem Mengenanteil von 0,1 bis 20 Gew.-% und vorzugsweise von 0,5 bis 10 Gew.-%, bezogen auf das Gesamtgewicht der Zusammensetzung, vorliegt.

**16.** Zusammensetzung nach einem der vorhergehenden Ansprüche, **dadurch gekennzeichnet, dass** das zweite organische Lösemittel einen Verteilungskoeffizienten D' von größer oder gleich 0,5 aufweist.

**17.** Zusammensetzung nach einem der vorhergehenden Ansprüche, **dadurch gekennzeichnet, dass** das zweite organische Lösemittel unter Propylenglykol-n-butylether, Dipropylenglykoldimethylether, Propylenglykolmethyletheracetat, Propylenglykolpropylether, Methyllactat, Ethyllactat, Isopropyllactat und den Gemischen dieser Verbindungen ausgewählt ist.

**18.** Zusammensetzung nach einem der vorhergehenden Ansprüche, **dadurch gekennzeichnet, dass** das zweite organische Lösemittel unter Propylenglykol-n-butylether, Dipropylenglykoldimethylether, Isopropyllactat und den Gemischen dieser Verbindungen ausgewählt ist.

**19.** Zusammensetzung nach einem der vorhergehenden Ansprüche, **dadurch gekennzeichnet, dass** das zweite organische Lösemittel in einem Mengenanteil von 2 bis 15 Gew.-% und vorzugsweise von 3 bis 10 Gew.-%, bezogen auf das Gesamtgewicht der Zusammensetzung, vorliegt.

**20.** Zusammensetzung nach einem der vorhergehenden Ansprüche, **dadurch gekennzeichnet, dass** sie ferner mindestens einen Zusatzstoff enthält, der unter Verdickungsmitteln, Mitteln zur Verteilung, Netzmitteln, Dispergiermitteln, Mitteln gegen Schaumbildung, Konservierungsstoffen, UV-Filtern, Färbemitteln, Pigmenten, Wirkstoffen, grenzflächenaktiven Stoffen, Hydratisierungsmitteln, Parfums, Neutralisationsmitteln, Stabilisierungsmitteln und Antioxidantien ausgewählt ist.

**21.** Kosmetisches Verfahren zum Schminken und/oder zur Pflege der Nägel, das darin besteht, eine Zusammensetzung nach einem der Ansprüche 1 bis 20 auf die Nägel aufzubringen.

**22.** Verwendung eines Acrylpolymers in wässeriger Dispersion, das eine Glasübergangstemperatur (Tg) von größer oder gleich 45 °C und eine minimale Temperatur für die Filmbildung (TMF) aufweist, die so ist, dass Tg - TMF ≤ 20 °C ist, eines ersten organischen Lösmittels mit einem bei atmosphärischem Druck bestimmten Siedepunkt von größer oder gleich 225 °C und eines zweiten organischen Lösemittels mit einem bei atmosphärischem Druck bestimmten Siedepunkt im Bereich von 70 bis 180 °C in einer Zusammensetzung zum Lackieren oder zur Pflege der Nägel, um einen Film zu erhalten, der mit Aceton und/oder Ethylacetat abgenommen werden kann und/oder auf dem Nagel haftet und/oder glänzt.